# EUROPEAN PATENT SPECIFICATION

(11) **EP 4 338 709 B1**
(45) Date of publication and mention of the grant of the patent: **07.01.2026**
(21) Application number: 22196026.3
(22) Date of filing: 16.09.2022
(51) Int. Cl.: A61F 2/38, A61F 2/30

(54) **POSTERIOR STABILIZED KNEE PROSTHESIS SYSTEM**
POSTERIOR STABILISIERTE KNIEPROTHESE
SYSTÈME DE PROTHÈSE DE GENOU POSTÉRO-STABILISÉ

(43) Date of publication of application: 20.03.2024
(73) Proprietor: Aesculap AG, 78532 Tuttlingen (DE)
(72) Inventor: Richter, Berna, 78570 Mühlheim (DE); Blanc, Steeven, 52000 Chaumont (FR); Lang, Rachel, 52000 Chaumont (FR); Grupp, Thomas, 78588 Denkingen (DE)
(74) Representative: Patentanwälte Ruff, Wilhelm, Beier, Dauster & Partner mbB

(56) References cited:
- US-A1- 2010 161 067
- US-A1- 2014 142 713
- US-A1- 2022 008 208
- US-B1- 6 699 291

## Description

The invention relates to a posterior stabilized knee prosthesis system according to claim 1.

Total knee arthroplasty (TKA) is a well-known surgical procedure by which a patient's deceased and/or damaged natural knee joint is replaced by a knee prosthesis. Typical knee prostheses are designed to replicate the movement of the patient's natural joint over the full range of motion (ROM), throughout and between full flexion and extension and in all planes (coronal-varus/valgus, sagittal-flexion, transverse-rotation).

Typical knee prostheses comprise a femoral component and a tibial component configured to be secured to a surgically-prepared distal end of a patient's femur and a surgically-prepared proximal end of the patient's tibia, respectively. The femoral component typically comprises a condyle surface with multiple tangential radii of curvature. The tibial component typically comprises a bearing surface with multiple tangential radii of curvature. As the knee is flexed and extended, the condyle surface and the bearing surface articulate and undergo combinations of relative anterior-posterior motion and relative internal-external rotation.

The type of knee prosthesis used may also depend on whether a patient's posterior cruciate ligament (PCL) is retained or sacrificed, i.e., removed, during surgery. A removal of the PCL may be necessary in case of insufficiency due to disease and/or damage. If the PCL is removed, posterior stabilized knee prostheses are oftentimes used and intend to provide additional support and/or control of the knee movement by replicating the function of the natural PCL. Typical posterior stabilized knee prostheses comprise a posterior cam on the femoral component and a post on the tibial component. The cam and the post typically intend to engage at a degree of flexion that resembles the degree of flexion when the native PCL would start its action.

To accommodate for different patients' anatomies, in particular for different femoral and tibial bone sizes, differently sized femoral and tibial components are provided. Said differently sized components form a knee prosthesis system. During surgery the surgeon decides which component sizes fit best. Due to the patient's anatomy or other surgical reasons it may be necessary to combine different sizes of femoral and tibial components of the knee prosthesis system.

EP 2 726 020 B1 discloses a posterior stabilized knee prosthesis system having the features specified in the preamble of claim 1, wherein the radii of curvature of the condyle surfaces decrease gradually between early-flexion and mid-flexion and increase during mid-flexion. Moreover, EP 2 726 020 B1 teaches that at least one radius of the radii of the condyle surfaces decreases over increasing femoral component size, while other radii increase.

US 6 699 291 B1 discloses another posterior stabilized knee prosthesis system according to the preamble of claim 1.

US 2014/142713 A1 discloses a knee prosthesis system, with femoral components of different sizes, the differently sized femoral components having condyle surfaces with different radii, wherein some of the radii increase across increasing size, and wherein at least one radius of the different radii decreases across increasing size. The femoral components of said prior art system do not comprise a posterior cam. Tibial components of said prior art system do not comprise a post.

### SUMMARY OF THE INVENTION

It is an object of the invention to provide a posterior stabilized knee prosthesis system of the type mentioned at the beginning, which allows for an improved prosthetic kinematic behavior.

This object is solved by providing a posterior stabilized knee prosthesis system with the features of claim 1. Preferred embodiments are defined in the dependent claims.

According to the invention, the radii of curvature of the condyle surface each increase monotonically across increasing size of the femoral components, and the radii of curvature of the bearing surface each increase monotonically across increasing size of the tibial components.

The invention reduces size dependent effects on the prosthesis' kinematic behavior. The inventors have found that the monotonic increase of the radii of curvature of the condyle surface and the radii of curvature of the bearing surface of the differently sized femoral and tibial components leads to a more predictable, homogeneous kinematic behavior regarding different same-sized combinations of femoral and tibial components as well as off-sized component combinations. In particular, the invention allows for a similar, and therefore predictable roll-back behavior for same-sized and off-sized component combinations.

The set of femoral components comprises at least three, preferably at least five, more preferably at least nine, different sizes. The same applies mutatis mutandis for the set of tibial components. The term "size" refers to a value within a sizing system and/or metric. A same-sized combination of components refers to a combination of a femoral component and a tibial component being assigned to the same value within said sizing system and/or metric. "Same-sized" does not mean that a given combination of femoral and tibial components has the same component dimensions. An "off-sized" combination of components refers to a given combination of a femoral component and a tibial component having different component sizes.

The condyle surfaces of the differently sized femoral components are each having said multiple radii, for example at least a first radius, a second radius and a third radius. According to the invention each radius increases monotonically over increasing femoral component size, for example a (small) first size, a (medium) second size and a (large) third size. "Monotonic" or "monotone" means that said radii are not decreasing over increasing femoral component size. Hence, because of the monotonic increase, the first radius of the third size femoral component is larger than or equal to the first radius of the second size femoral component. In turn, the first radius of the second size femoral component is larger than or equal to the first radius of the first size femoral component. Moreover, the second radius of the third size femoral component is larger than or equal to the second radius of the second size femoral component. In turn, the second radius of the second size femoral component is larger than or equal to the second radius of the first size femoral component. The same applies mutatis mutandis regarding the third radii. Put in other words: From one size to a larger femoral component size none of the radii decreases. The multiple radii are each uniform and/or discrete, i.e., each radius - for a given size of femoral component - has a fixed non-gradual value. In embodiments, the multiple radii of the condyle surface are tangential. Preferably, the multiple radii of the condyle surface are positioned posterior to a dwell point of the condyle surface and/or posterior to an anterior radius. The multiple radii of the condyle surface can therefore be referred to as posterior radii.

The bearing surface of the differently sized tibial components are each having said multiple radii of curvature, for example a first radius and a second radius. According to the invention, each radius of the bearing surface increases monotonically over increasing tibial component size, for example for a (small) first size, a (medium) second size and a (large) third size. As a result of the monotonic increase, the first radius of the third size tibial component is larger than or equal to the first radius of the second size tibial component. In turn, the first radius of the second size tibial component is larger than or equal to the first radius of the first size tibial component. Moreover, the second radius of the third size tibial component is larger than or equal to the second radius of the second size tibial component. Moreover, the second radius of the second size tibial component is larger than or equal to the second radius of the first size tibial component. Put in other words, none of the radii of the bearing surfaces decreases with increasing size of the tibial components. The multiple radii of the bearing surfaces are each uniform and/or discrete, i.e., each radius - for a given size of tibial component - has a fixed non-gradual value. In embodiments, the multiple radii of the bearing surfaces are tangential. Preferably, the bearing surfaces each have a first radius and a second radius, wherein the first radius is an anterior radius positioned anterior to a dwell point of the respective bearing surface and the second radius is a posterior radius positioned posterior to said dwell point.

In one embodiment, the increase of the radii of curvature of the condyle surface across increasing size of the femoral components is strictly monotonic and/or the increase of the radii of curvature of the bearing surface across increasing size of the tibial components is strictly monotonic. Hence, the respective radii are strictly increasing with increasing size of the femoral components or the tibial components, respectively. In this embodiment therefore a radius on a larger size femoral component is always larger than the respective radius on a smaller size femoral component. The same applies mutatis mutandis with respect to larger and smaller size tibial components and their respective radii. In embodiments, the strictly monotonic increase regarding the radii of the condyle surfaces is at least substantially proportional, i.e., linear, progressive, in particular exponential, and/or degressive with increasing femoral component size. The same applies mutatis mutandis with respect to the strictly monotonic increase of the radii of the bearing surfaces.

In one embodiment, the radii of curvature of the condyle surface each increase at least substantially linearly across increasing size of the femoral components and/or the radii of curvature of the bearing surface each increase at least substantially linearly across increasing size of the tibial components. The inventors have found that said at least substantially linear increase leads to particularly advantageous kinematic behavior for same-sized as well as off-sized component combinations.

In one embodiment, the condyle surface of each femoral component has a femoral dwell point, wherein an anterior-posterior distance between the femoral dwell point and an anterior edge of the condyle surface increases, preferably strictly, monotonically across increasing size of the femoral components. The femoral dwell point is the most distal point of the respective condyle surface. The inventors have found that the, in particular strictly, monotonic increase of the anterior-posterior distance between the femoral dwell point and the anterior edge of the respective condyle surface (for each femoral component size) allows for further improvements in the prosthetic kinematic behavior. In particular, the kinematic behavior is less size dependent in comparison to designs featuring a fixed anterior-posterior distance for all sizes. In embodiments, the strictly monotonic increase is at least substantially proportional, i.e., linear, progressive, in particular exponential, and/or degressive with increasing tibial component size.

In one embodiment, the anterior-posterior distance between the femoral dwell point and the anterior edge of the condyle surface increases at least substantially linearly across increasing size of the femoral components. Given that a total anterior-posterior dimension of the femoral components increases at least substantially linearly across increasing component size, the at least substantially linearly increase of the anterior-posterior distance corresponds to a fixed percentage of said total anterior-posterior dimension. In embodiments, the increase of the anterior-posterior distance is linear across increasing size of the femoral components.

In one embodiment, the anterior-posterior distance is between 55% and 65%, preferably 60%, of a total anterior-posterior dimension of the respective femoral component. The inventors have found that 60% is an at least nearly optimal value regarding the resulting kinematic behavior.

In one embodiment, the bearing surface of each tibial component has a tibial dwell point, wherein an anterior-posterior distance between the tibial dwell point and an anterior edge of the bearing surface increases, preferably strictly, monotonically across increasing size of the tibial components. The tibial dwell point is the most distal point of the bearing surface of the respective tibial component. The, preferably strictly, monotonic increase of the anterior-posterior distance between the tibial dwell point and the anterior edge of the respective bearing surface leads to further improvements in the kinematic behavior. In embodiments, the strictly monotonic increase is at least substantially proportional, i.e., linear, progressive, in particular exponential, and/or degressive with increasing tibial component size.

In one embodiment, the anterior-posterior distance between the tibial dwell point and an anterior edge of the bearing surface increases at least substantially linearly across increasing size of the tibial components. Given that a total anterior-posterior dimension of the tibial components increases at least substantially linearly across increasing size of the tibial components. This corresponds to an at least substantially fixed percentage of the anterior-posterior distance with respect to the total anterior-posterior dimension of the respective tibial component.

In one embodiment, the anterior-posterior distance is between 60% and 70%, preferably 65%, of a total anterior-posterior dimension of the respective tibial component. The inventors have found that 65% is an at least nearly optimal value regarding the resulting kinematic behavior. In embodiments, an anterior-posterior position of the post in relation to the tibial dwell point has a fixed value over increasing size of the tibial components. Hence, the position of the post evolves smoothly over increasing size of the tibial components. In particular, a percentage of the position of the post decreases linearly over increasing size of the tibial components. The inventors have found that this may lead to a further improvement of the kinematic behavior. In particular, size dependencies for both same-sized and off-sized component combinations can be further reduced.

In one embodiment, the multiple at least substantially tangential radii of curvature of the condyle surface of each femoral component decrease, preferably strictly, monotonically in posterior direction along the condyle surface. In contrast to prior art designs featuring at least one increasing radius in posterior direction along the condyle surface, this embodiment may lead to a further improved kinematic behavior, in particular during a movement between extension and full flexion.

In one embodiment, the condyle surface of each femoral component has a first curved surface section with a first radius of curvature contacting the bearing surface during flexion between extension and a first degree of flexion, and a second curved surface section with a second radius of curvature contacting the bearing surface during flexion between the first degree of flexion and a larger second degree of flexion. Preferably, in extension the femoral dwell point and the tibial dwell point of the respective components of a given component combination contact each other. The first curved surface section is positioned posterior from the femoral dwell point. The second curved surface section is positioned posterior from the first curved surface section. Preferably the first radius and/or the second radius is unitary and/or discrete, i.e., does not change its value along the respective surface section.

In one embodiment, a ratio of the first radius of curvature to the second radius of curvature decreases, preferably strictly, monotonically across increasing size of the femoral components. In comparison to prior art designs featuring a substantially constant or slightly increasing ratio, this embodiment may lead to further improvements regarding the kinematic behavior.

In one embodiment, the ratio of the first radius of curvature to the second radius of curvature decreases in the range of 1.380 to 1.240. Hence, the ratio is 1.380 for the smallest size femoral component and 1.240 for the largest size femoral component of the set of femoral components.

In one embodiment, the condyle surface of each femoral component has a third curved surface section with a third radius of curvature contacting the bearing surface during flexion between the second degree of flexion and a larger third degree of flexion, wherein a ratio of the second radius of curvature to the third radius of curvature decreases, preferably strictly, monotonically across increasing size of the femoral components. The third curved surface section is positioned posterior in relation to the second curved surface section. In comparison to prior art designs featuring an increasing or nearly linear ratio of the second to the third radius of curvature, this embodiment may lead to a further improved kinematic behavior.

In one embodiment, the ratio of the second radius of curvature to the third radius of curvature decreases in the range of 1.031 to 1.019. Hence, the ratio of the second to the third radius of curvature decreases moderately over increasing femoral component size. Moreover, there is only a slight decrease from the second to the third radius on a given femoral component. Put in other words, the "gap" between the second radius and the third radius is less abrupt than in some prior art designs, which allows for a more linear/smooth kinematic behavior during flexion between the second and the third degree of flexion. In embodiments, the ratio for the smallest femoral component is 1.031 and 1.019 for the largest femoral component of the set of femoral components.

In one embodiment, the condyle surface of each femoral component has a fourth curved surface section with a fourth radius of curvature contacting the bearing surface during flexion between the third degree of flexion and a larger fourth degree of flexion, wherein a ratio of the third radius of curvature to the fourth radius of curvature decreases, preferably strictly, monotonically across increasing size of the femoral components. The fourth curved surface section is posterior in relation to the third curved surface section. In comparison to prior art designs featuring a nearly constant or increasing ratio of the third radius to the fourth radius, this embodiment may result in further improvements of the kinematic behavior.

In one embodiment, the ratio of the third radius of curvature to the fourth radius of curvature decreases in the range of 1.059 to 1.036. The ratio is 1.059 for the smallest size and 1.036 for the largest size femoral component of the set of femoral components. Hence, for both components, the smallest and the largest as well as for those components in-between, the "gap" between the third and the fourth radius is relatively small and/or less abrupt than in some prior art designs.

In one embodiment, the condyle surface of each femoral component has a fifth curved surface section with a fifth radius of curvature contacting the bearing surface during flexion between the fourth degree of flexion and a larger fifth degree of flexion, wherein a ratio of the fourth radius of curvature to the fifth radius of curvature decreases, preferably strictly, monotonically across the size of the femoral components. In comparison to prior art designs featuring a nearly constant or increasing ratio of the fourth to the fifth radius of curvature, this embodiment may lead to further improvements regarding the kinematic behavior. The fifth curved surface section is posterior in relation to the fourth curved surface section.

In one embodiment, the ratio of the fourth radius of curvature to the fifth radius of curvature decreases in the range of 1.020 to 1.012. As a result, there is only a very small "gap" between the fourth and fifth radius for all femoral component sizes. Put in other words, the transition from the fourth to the fifth radius is smaller than in some prior art designs. This may lead to a more linear/smooth kinematic behavior during an articulation of the fourth and fifth curved surface section with the bearing surface. In embodiments, the ratio is 1.020 for the smallest size and 1.012 for the largest size femoral component of the set of femoral components.

In one embodiment, a ratio of the first radius of curvature to the fifth radius of curvature decreases, preferably strictly, monotonically in the range of 1.537 to 1.326. Preferably, the fifth radius of curvature is the most posterior and/or the last radius on the respective condyle surface. Hence, the difference or "gap" between the first radius and the fifth, in particular last, radius is less than in some prior art designs. As a result, the condyle surfaces each have a rounder, less oval shape. This may lead to further improved kinematic properties. In embodiments, the ratio of the first to the fifth radius is 1.537 for the smallest and 1.326 for the largest component of the set of femoral components.

In one embodiment, the cam initially engages the post at a degree of flexion between 35° and 60°, preferably between 45° and 60°. As a result, initial cam-post engagement takes place at a degree of flexion that corresponds to a degree of flexion when a native PCL starts to act in a native knee. In embodiments, the engagement degree of flexion is smaller than the first degree of flexion. In other embodiments, the engagement degree of flexion is larger than the first degree of flexion and smaller than the second degree of flexion. In yet other embodiments, the engagement degree of flexion is larger than the second degree of flexion and smaller than the third degree of flexion. In still other embodiments, the engagement degree of flexion is larger than the third degree of flexion and smaller than the fourth degree of flexion. Preferably, the engagement degree of flexion is larger than the second degree of flexion and smaller than the third degree of flexion.

### BRIEF DESCRIPTION OF THE DRAWINGS

In the following, a preferred embodiment of the invention is described in detail with reference to the drawings. Throughout the drawings, same elements are denoted with same reference numerals/signs. The drawings schematically show:
- fig. 1: a perspective view of an embodiment of a posterior stabilized knee prosthesis system according to the invention, the knee prosthesis system having a set of differently sized femoral components and a set of differently sized tibial components;
- fig. 2: a perspective view of a femoral component of the knee prosthesis system according to fig. 1;
- fig. 3: a perspective view of a tibial component of the knee prosthesis system according to fig. 1;
- fig. 4: a perspective view of a knee prosthesis comprising the femoral and tibial components according to figs. 2 and 3;
- fig. 5: a side view of the femoral component according to fig. 2;
- fig. 6: another perspective view of the tibial component according to fig. 3;
- fig. 7: a table containing a set of total anterior-posterior dimension values and a set of radii of curvature values for the set of differently sized femoral components;
- fig. 8: a table containing the set of total anterior-posterior dimensions and a set of degree of flexion values for the set of differently sized femoral components;
- fig. 9: a graph showing the increase of the radii contained in the table of fig. 7 over the size of the femoral components;
- fig. 10: a table containing a set of total anterior-posterior dimension values and a set of radii of curvature values for the set of differently sized tibial components;
- fig. 11: a graph showing the radii of curvature values contained in the table of fig. 10 over increasing tibial component size;
- fig. 12: a graph of a relative anterior-posterior position of a femoral dwell point over the total anterior-posterior dimension values of the differently sized femoral components as contained in the table of figs. 7;
- fig. 13: a graph of a relative anterior-posterior position of a tibial dwell point over the total anterior-posterior dimension values of the tibial components as contained in the table of fig. 10;

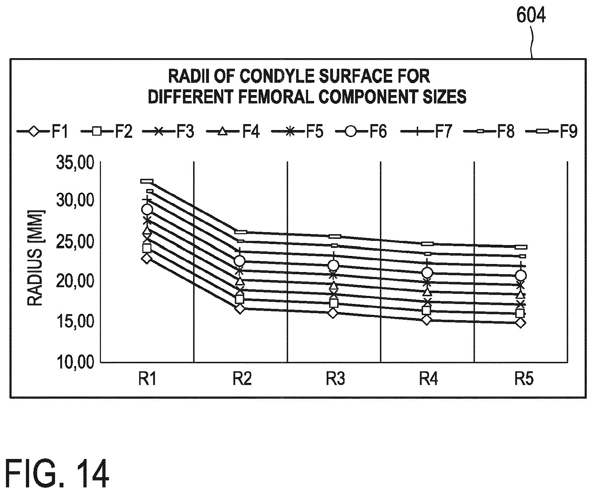

- fig. 14: a graph of the radii of curvature values contained in the table of fig. 7;
- fig. 15: a table containing the set of total anterior-posterior dimension values and a set of ratios of the radii of curvature values contained in the table of fig. 7;
- fig. 16: a graph of the ratios of radii contained in the table of fig. 15 over increasing femoral component size;
- fig. 17: a graph of a roll-back movement over a degree of flexion for the differently sized femoral components and
- fig. 18: a graph of a roll-back movement over the degree of flexion for differently sized femoral components according to a prior art design.

### DETAILED DESCRIPTION OF AN EXEMPLARY EMBODIMENT OF THE INVENTION

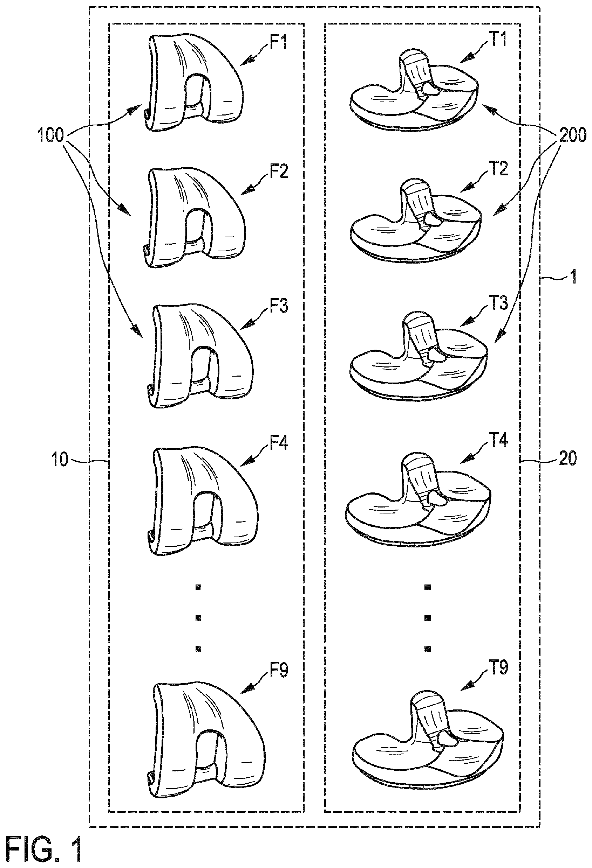

According to fig. 1, a posterior stabilized knee prosthesis system 1 comprises a set 10 of femoral components 100 of different sizes F1 to F9 and a set 20 of tibial components 200 of different sizes T1 to T9. The differently sized femoral components 100 are configured for attachment to distal femurs of different sizes. The differently sized tibial components 200 are configured for attachment to proximal tibiae of different sizes. Depending on a patient's anatomy, in particular the femoral and tibial bone sizes, the differently sized femoral and tibial components 100, 200 are configured for same-size and off-size combination to form a knee prosthesis for the replacement of the patient's natural knee joint. Put in other words, each size F1 to F9 of femoral component 100 is engageable to at least one size T1 to T9 of tibial component 200 and vice versa.

Apart from their different sizes F1 to F9, the femoral components 100 of the set 10 have an identical design. The same applies mutatis mutandis to the tibial components 200 of the set 20.

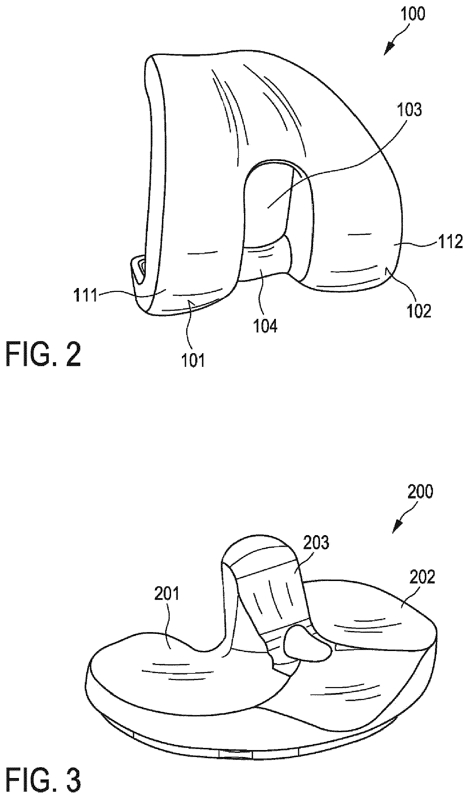

Further features of the femoral components 100 and the tibial components 200 will be described with reference to figs. 2 to 6 and regarding a single femoral component 100 and a single tibial component 200 of the set 10 and the set 20, respectively, well knowing that the remaining components of said sets 10, 20 have identical features apart from their different sizes.

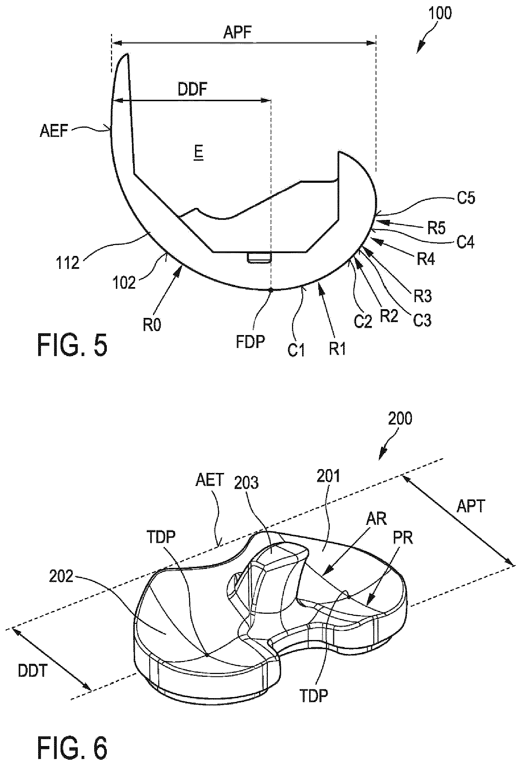

Referring to fig. 2, the femoral component 100 has a pair of spaced apart condyles 111, 112. The condyles 111, 112 are spaced apart in medial-lateral direction and define an intercondylar notch 103. A posterior cam 104 is positioned in the intercondylar notch 103. The condyles 111, 112 can be referred to as medial condyle 111 and lateral condyle 112 or vice versa, depending on whether the femoral component 100 is used on the patient's left or right distal femur. Both condyles 111, 112 comprise a condyle surface 101, 102, referred to as medial condyle surface 101 and lateral condyle surface 102. Both condyle surfaces 101, 102 are curved in the sagittal plane E (see fig. 5). In the embodiment shown, the condyle surfaces 101, 102 are at least substantially symmetrical with respect to a sagittal symmetry plane, while other embodiments comprise non-symmetrical condyle surfaces. Further features of the condyle surfaces 101, 102 will be described with reference to the lateral condyle surface 102 and fig. 5. Due to the symmetrical design of the condyle surfaces 101, 102 the further features described with respect to the lateral condyle surface 102 apply mutatis mutandis to the medial condyle surface 101.

Referring to fig. 5, the (lateral) condyle surface 102 is curved in the sagittal plane E with multiple at least substantially tangential radii R0, R1 to R5 of curvature. The radius R0 is located anterior to a femoral dwell point FDP of the condyle surface 102. The radii R1 to R5 are located posterior to the dwell point FDP. The femoral dwell point FDP denotes the most distal point of the condyle surface 102 and/or the condyle 112. The radius R0 can therefore also be denoted as anterior radius R0. The radii R1 to R5 can also be denoted as posterior radii of the femoral component 100.

Referring to fig. 3, the tibial component 200 comprises bearing surfaces 201, 202 that can also be denoted as medial bearing surface 201 and lateral bearing surface 202 or vice versa, depending on whether the tibial component 200 is used on the patient's left or right proximal tibia. The bearing surfaces 201, 202 are spaced apart in medial-lateral direction. The tibial component 200 further comprises a post 203 extending upwardly, i.e., in proximal direction, from the bearing surfaces 201, 202.

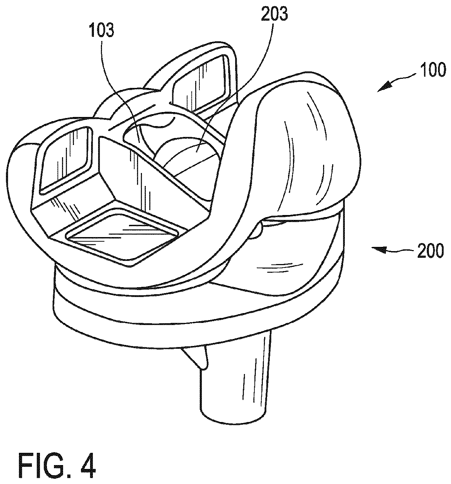

The femoral component 100 and the tibial component 200 are configured to articulate by contact between the condyle surfaces 101, 102 and the bearing surfaces 201, 202 (see fig. 4). The components 100, 200 are also configured to articulate by contact between the cam 104 and the post 203, the cam 104 and the post 203 not being engaged throughout the full range of motion of the knee prosthesis.

In the embodiment shown, the bearing surfaces 201, 202 are symmetrical with respect to a sagittal symmetry plane, while other embodiments have non-symmetrical bearing surfaces. Further features of the bearing surfaces 201, 202 will be described with reference to fig. 6. Features described regarding the medial bearing surface 201 apply mutatis mutandis to the lateral bearing surface 201 and vice versa.

The (medial) bearing surface 201 is curved in the sagittal plane E (see fig. 5) with multiple at least substantially tangential radii AR, PR of curvature. The bearing surface 201 comprises a tibial dwell point TDP denoting the most distal point on the bearing surface 201. The radius AR is positioned anterior to the tibial dwell point TDP and can therefore be denoted as anterior radius (of the tibial component 200). The radius PR is positioned posterior to the tibial dwell point TDP and can therefore be denoted as posterior radius PR (of the tibial component 200).

In full extension of the knee prosthesis depicted in fig. 4 the contact between the femoral component 100 and the tibial component 200 is established between the femoral dwell point FDP and the tibial dwell point TDP, more precisely: between the respective dwell points on the medial and lateral surfaces, respectively. When the knee prosthesis is flexed, the "point" of contact moves along the posterior radii R1 to R5 of the femoral component 100 and the posterior radius PR of the tibial component 200.

In the embodiment shown, the cam 104 initially engages the post 203 at a degree of flexion between 35° and 60°, more precisely between 45° and 60°. The range of 45° to 60° corresponds to the degree of flexion when a native posterior cruciate ligament starts to action in a native knee.

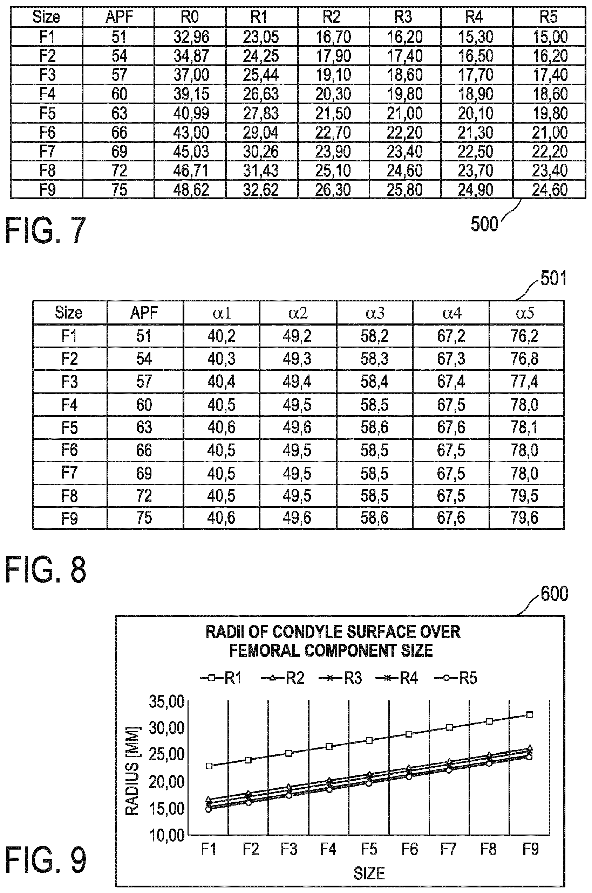

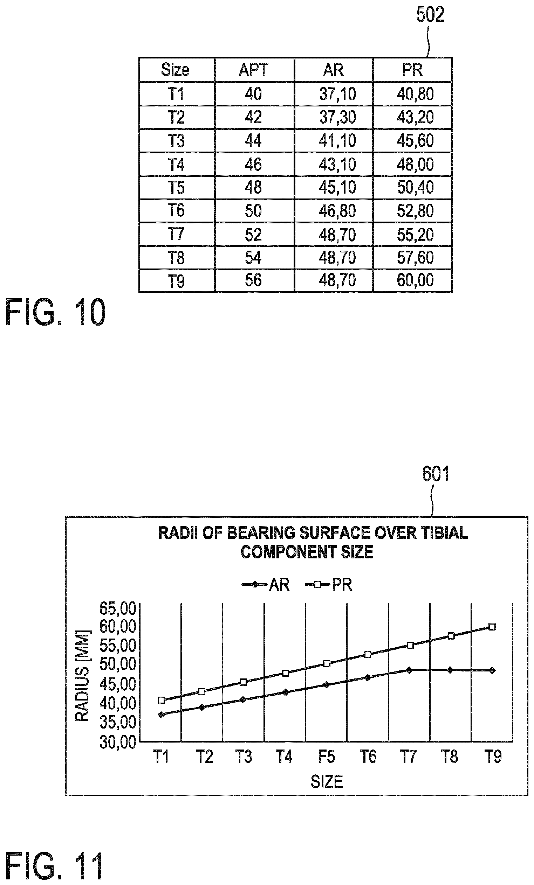

As can be seen from table 500 shown in fig. 7, the radii R0, R1 to R5 of the condyle surfaces 101, 102 each increase monotonically across increasing size F1 to F9 of the femoral components 100. Moreover, the radii AR, PR of curvature of the bearing surfaces 201, 202 each increase monotonically across increasing size T1 to T9 of the tibial components (see table 502 shown in fig. 10).

In the embodiment shown, F1 is the smallest and F9 is the largest size of the femoral components 100. Correspondingly, T1 is the smallest and T9 is the largest size of the tibial components 200. The total number of sizes, in this case nine femoral and nine tibial sizes, is purely exemplary. In other embodiments, the set 10 has less or more than nine femoral sizes, for example two, three, four, five, six, seven, eight, ten, eleven, twelve or even more sizes. The same applies mutatis mutandis with respect to the number of tibial sizes.

Referring to figs. 5 and 7, the radii R1 to R5 can be denoted as first radius R1, second radius R2, third radius R3, fourth radius R4 and fifth radius R5 of curvature of the condyle surface 101, 102. In other embodiments, the condyle surfaces 101, 102 comprise less or more than five (posterior) radii, for example two, three, four, six, seven, eight or even more radii. Referring to figs. 6 and 10, the same applies mutatis mutandis with respect to the radii AR, PR of the bearing surfaces 201, 202.

Because of the monotonic increase of the radii R0, R1 to R5 as well as AR and PR, the respective radius increases or stays the same with increasing size F1 to F9 of the femoral component 100 and increasing size T1 to T9 of the tibial component 200, respectively.

In the embodiment shown, the radii R0, R1 to R5 of the femoral components 100 increase strictly monotonically (see table 500). A strictly monotonic or monotone increase means that each of the radii R0, R1 to R5 increases starting from the smallest size F1 to every further size F2 to F8 until the largest size F9. In the embodiment shown, the same applies mutatis mutandis to the increase of the posterior radius PR of the bearing surfaces 201, 202 (see table 502 of fig. 10). Hence, the posterior radius PR increases from the smallest size T1 to every further size T2 to T8 until the largest size T9 of the tibial components 200. The anterior radius AR, however, does not increase strictly monotonically over the full range of sizes T1 to T9. With reference to table 502, it is evident that the anterior radius AR has equal values for the sizes T7, T8, T9. In other embodiments, also the anterior radius AR increases strictly monotonically.

In the embodiment shown, the radii R1 to R5 of the condyle surfaces 101, 102 each increase linearly across increasing size F1 to F9 of the femoral components 100. Said linear increase is shown in graph 600 of fig. 9. Referring to graph 601 of fig. 11, the same applies mutatis mutandis regarding the posterior radius PR. The graph 601 shows that the posterior radius PR of the bearing surfaces 201, 202 increases linearly across increasing size T1 to T9 of the tibial components 200. The anterior radius AR, however, does not increase linearly across the full range of sizes T1 to T9. Instead, the increase is linear in-between the sizes T1 and T7, while there is no increase from size T7 to size T8 and from size T8 to size T9.

In other embodiments, the afore-mentioned strictly monotonic increases are not linear, but instead progressive, in particular exponential, and/or degressive with increasing component size.

Further with reference to fig. 5, the femoral component 100 has a total anterior-posterior dimension APF extending between an anterior edge AEF and a posterior edge (without reference sign). The total anterior-posterior dimension APF increases linearly over increasing femoral component size F1 to F9 (see column 2 of table 500 in fig. 7). Due to the proportional dependence between femoral component size F1 to F9 and total anterior-posterior dimension APF, the linear increase of the radii R1 to R5 shown in graph 600 of fig. 7 is also linear over increasing total anterior-posterior dimension APF.

Referring to fig. 6, the tibial component 200 has a total anterior-posterior dimension APT extending between an anterior edge AET and a posterior edge (without reference sign). As can be seen from the values in column 2 of table 502 of fig. 10, the total anterior-posterior dimension APT of the tibial component 200 increases linearly over increasing tibial component size T1 to T9. As a result, the linear increase of the posterior radius PR illustrated in graph 601 of fig. 11 is linear over increasing total anterior-posterior dimension APT of the tibial components 200 as well.

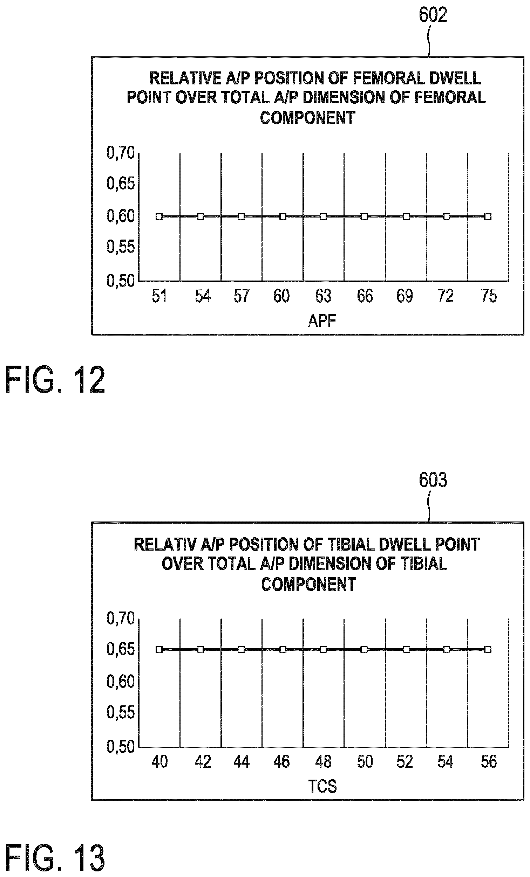

Further referring to fig. 5, the femoral dwell point FDP is positioned at an anterior-posterior distance DDF from the anterior edge AEF. The anterior-posterior distance DDF between the femoral dwell point FDP and the anterior edge AEF increases monotonically across increasing femoral component size F1 to F9. More precisely, in the embodiment shown, said increase is linear. As a result, a ratio between the total anterior-posterior dimension APF and the anterior-posterior distance DDF is constant for all femoral component sizes F1 to F9 (see graph 602 of fig. 12). In the embodiment shown, said ratio, i.e., a relative anterior-posterior position of the femoral dwell point DDF, is 0.60. Put in other words, the anterior-posterior distance DDF is 60% of the total anterior-posterior dimension APF for all femoral component sizes F1 to F9. In other embodiments, said ratio is between 0.55 and 0.65.

Referring to fig. 6, the tibial dwell point TDP is positioned in an anterior-posterior distance DDT from the anterior edge AET of the tibial component 200. Said distance DDT increases monotonically across increasing tibial component size T1 to T9. More precisely, in the embodiment shown, said increase is linear. Since the total anterior-posterior dimension APT is proportional to the tibial component size, a ratio between the anterior-posterior distance DDT and the total anterior-posterior dimension APT stays constant for all tibial component sizes T1 to T9. Said ratio, i.e., a relative anterior-posterior position of the tibial dwell point TDP, is shown in graph 603 of fig. 13. In the embodiment shown, said ratio is 0.65. Put in other words, the anterior-posterior distance DDT is 65% of the total anterior-posterior dimension APT for all tibial component sizes T1 to T9. In other embodiments the relative value is between 60% and 70%.

Again, with reference to fig. 5 and table 500 of fig. 7, it is evident that the radii R0, R1 to R5 of the condyle surfaces 101, 102 - for each size F1 to F9 - decrease in posterior direction along the condyle surface 101, 102. Put in other words, the anterior radius R0 is larger than the first radius R1, the first radius R1 is larger than the second radius R2, the second radius R2 is larger than the third radius R3, the third radius R3 is larger than the fourth radius R4 and the fourth radius R4 is larger than the fifth radius R5. Afore-mentioned relations apply for every femoral component size F1 to F9. Since the order of radii along the posterior direction of the condyle surfaces 101, 102 is "R0", "R1", "R2", "R3", "R4", "R5", the values of the radii decrease in posterior direction.

Starting from full extension, i.e., a contact between the femoral dwell point FDP and the tibial dwell point TDP, the contact "point" between the femoral component 100 and the tibial component 200 moves along different curved surface sections C1 to C5 of the (lateral) condyle surface 102 (see fig. 5) and analogously the (medial) condyle surface 101.

In the embodiment shown, a first curved surface section C1 has the first radius R1, a second curved surface section C2 has the second radius R2, a third curved surface section C3 has the third radius R3, a fourth curved surface section C4 has the fourth radius R4 and a fifth curved surface section C5 has the fifth radius R5.

The first curved surface section C1 contacts the respective bearing surface 201, 202 during flexion between (full) extension and a first degree of flexion α1 (see column 3 of table 501 in fig. 8). The second curved surface section C2 is in contact between the first degree of flexion α1 and a larger second degree of flexion α2 (see column 4 of table 501). The third curved surface section C3 is in contact between the second degree of flexion α2 and a larger third degree of flexion α3 (see column 5 of table 501). The fourth curved surface section C4 is in contact between the third degree of flexion α3 and a larger fourth degree of flexion α4 (see column 6 of table 501). The fifth curved surface section C5 is in contact between the fourth degree of flexion α4 and a larger fifth degree of flexion α5 (see last column of table 501).

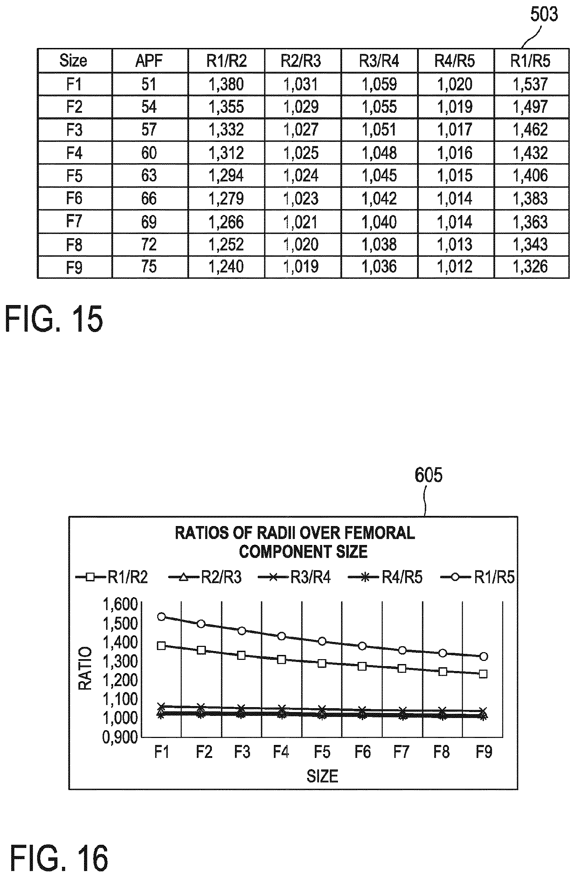

Table 503 of fig. 15 contains values for different ratios between the radii R1 to R5 for the set of femoral component sizes F1 to F9. A first ratio R1/R2 denotes the ratio between the first radius R1 and the second radius R2. A second ratio R2/R3 denotes the ratio between the second radius R2 and the third radius R3. A third ratio R3/R4 denotes the ratio between the third radius R3 and the fourth radius R4. A fourth ratio R4/R5 denotes the ratio between the fourth radius R4 and the fifth radius R5. A fifth ratio R1/R5 denotes the ratio between the first radius R1 and the fifth radius R5. The values in table 503 show that each ratio decreases - at least slightly - over increasing femoral component size F1 to F9. In the embodiment shown, said decrease is strictly monotonic for each of the ratios R1/R2, R2/R3, R3/R4, R4/R5 and R1/R5.

Table 503 further shows that the difference between the first (posterior) radius R1 and the last radius, in the present embodiment the fifth radius R5, is relatively small. The difference is smaller than in some prior art designs and leads to a less oval, rounder shape of the condyle surface in the sagittal plane (see fig. 5). Further, table 503 shows that the difference between one radius to the next radius - and therefore the ratios R1/R2, R2/R3, R3/R4, R4/R5 - is relatively small. Hence, said ratios range at approximately 1.

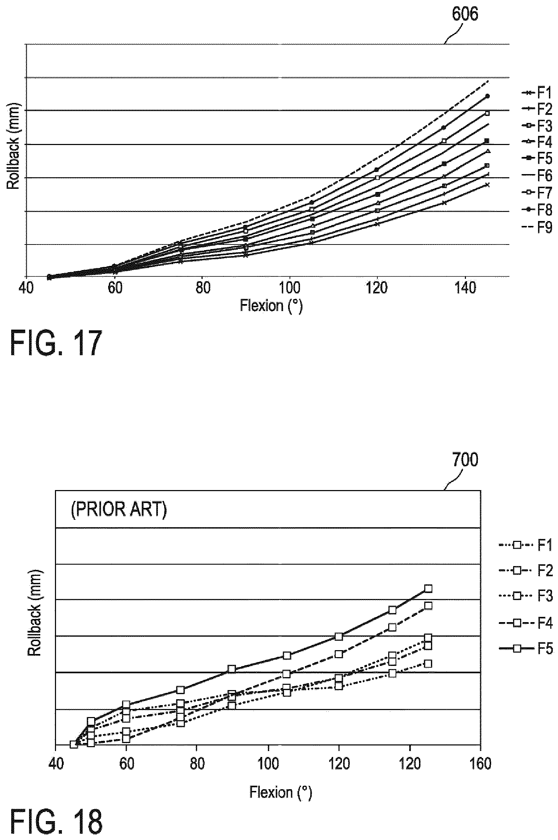

Fig. 17 shows a graph 606 illustrating the rollback movement during flexion for different femoral component sizes F1 to F9. As a result of the, in particular strictly, monotonic evolution of the design parameters, such as the radii R1 to R5 and their ratios, the evolution of the rollback is monotonic over increasing degrees of flexion and regarding the femoral component sizes F1 to F9. Put in other words, for a given degree of flexion, rollback is largest for the largest size F9 and smallest for the smallest size F1. For each size, the rollback increases monotonically over increasing degree of flexion.

In contrast to the rollback behavior illustrated in graph 606, graph 700 of fig. 18 illustrates the rollback behavior of a prior art design. Graph 700 shows that a monotonic increase of rollback over increasing femoral component size is not guaranteed for all degrees of flexion. Hence, the kinematic behavior of the prior art design illustrated in graph 700 is less predictable than the kinematic behavior illustrated by means of graph 606.

## Claims

1. A posterior stabilized knee prosthesis system (1), comprising:
a set (10) of femoral components (100) of different sizes (F1 to F9) configured for attachment to distal femurs of different sizes, each femoral component (100) having a pair of spaced apart condyles (111, 112) defining an intercondylar notch (103) therebetween, and having a posterior cam (104) positioned in the intercondylar notch (103), wherein at least one of the condyles (111, 112) has a condyle surface (101, 102) curved in the sagittal plane (E) with multiple at least substantially tangential radii (R0, R1 to R5) of curvature;
and a set (20) of tibial components (200) of different sizes (T1 to T9) configured for attachment to proximal tibiae of different sizes (T1 to T9), each tibial component (200) having a bearing surface (201, 202) curved in the sagittal plane (E) with multiple at least substantially tangential radii (AR, PR) of curvature, and having a post (203) extending upwardly from the bearing surface (201, 202);
wherein each size (F1 to F9) of femoral component is engageable to at least one size (T1 to T9) of tibial component to articulate by contact between the condyle surface (101, 102) and the bearing surface (201, 202) and/or by contact between the cam (104) and the post (203);
**characterized in that** the radii (R0, R1 to R5) of curvature of the condyle surface (101, 102) each increase monotonically across increasing size (F1 to F9) of the femoral components (100), and **in that** the radii (AR, PR) of curvature of the bearing surface (201, 202) each increase monotonically across increasing size (T1 to T9) of the tibial components (200).

2. The posterior stabilized knee prosthesis system (1) according to claim 1, wherein the increase of the radii (R0, R1 to R5) of curvature of the condyle surface (101, 102) across increasing size (F1 to F9) of the femoral components (100) is strictly monotonic and/or in that the increase of the radii (AR, PR) of curvature of the bearing surface (201, 202) across increasing size (T1 to T9) of the tibial components (200) is strictly monotonic.

3. The posterior stabilized knee prosthesis system (1) according to claim 1 or 2, wherein the radii (R0, R1 to R5) of curvature of the condyle surface (101, 102) each increase linearly across increasing size (F1 to F9) of the femoral components (100) and/or in that the radii (AR, PR) of curvature of the bearing surface (201, 202) each increase linearly across increasing size (T1 to T9) of the tibial components (200).

4. The posterior stabilized knee prosthesis system (1) according to any of the preceding claims, wherein the condyle surface (101, 102) of each femoral component (100) has a femoral dwell point (FDP), wherein an anterior-posterior distance (DDF) between the femoral dwell point (FDP) and an anterior edge (AEF) of the condyle surface (101, 102) increases, preferably strictly, monotonically across increasing size (F1 to F9) of the femoral components (100).

5. The posterior stabilized knee prosthesis system (1) according to claim 4, wherein the anterior-posterior distance (DDF) between the femoral dwell point (FDP) and the anterior edge (AEF) of the condyle surface (101, 102) increases linearly across increasing size (F1 to F9) of the femoral components (100).

6. The posterior stabilized knee prosthesis system (1) according to claim 4 or 5, wherein the anterior-posterior distance (DDF) is between 55% and 65%, preferably 60%, of a total anterior-posterior dimension (APF) of the respective femoral component (100).

7. The posterior stabilized knee prosthesis system (1) according to any of the preceding claims, wherein the bearing surface (201, 202) of each tibial component (200) has a tibial dwell point (TDP), wherein an anterior-posterior distance (DDT) between the tibial dwell point (TDP) and an anterior edge (AET) of the bearing surface (201, 202) increases, preferably strictly, monotonically across increasing size (T1 to T9) of the tibial components.

8. The posterior stabilized knee prosthesis system (1) according to claim 7, wherein the anterior-posterior distance (DDT) between the tibial dwell point (TDP) and the anterior edge (AET) of the bearing surface (201, 202) increases linearly across increasing size (T1 to T9) of the tibial components (200).

9. The posterior stabilized knee prosthesis system (1) according to claim 7 or 8, wherein the anterior-posterior distance (DDT) is between 60% and 70%, preferably 65%, of a total anterior-posterior dimension (APT) of the respective tibial component (200).

10. The posterior stabilized knee prosthesis system (1) according to any of the preceding claims, wherein the multiple at least substantially tangential radii (R0, R1 to R5) of curvature of the condyle surface (101, 102) of each femoral component (100) decrease, preferably strictly, monotonically in posterior direction along the condyle surface (101, 102).

11. The posterior stabilized knee prosthesis system (1) according to any of the preceding claims, wherein the condyle surface (101, 102) of each femoral component (100) has
a first curved surface section (C1) with a first radius (R1) of curvature contacting the bearing surface (201, 202) during flexion between extension and a first degree (α1), of flexion, and
a second curved surface section (C2) with a second radius (R2) of curvature contacting the bearing surface (201, 202) during flexion between the first degree (α1) of flexion and a larger second degree (α2) of flexion.

12. The posterior stabilized knee prosthesis system (1) according to claim 11, wherein a ratio (R1/R2) of the first radius (R1) of curvature to the second radius (R2) of curvature decreases, preferably strictly, monotonically across increasing size (F1 to F9) of the femoral components (100).

13. The posterior stabilized knee prosthesis system (1) according to claim 12, wherein the ratio (R1/R2) of the first radius (R1) of curvature to the second radius (R2) of curvature decreases in the range of 1.380 to 1.240.

14. The posterior stabilized knee prosthesis system (1) according to any of the claims 11 to 13, wherein the condyle surface (101, 102) of each femoral component (100) has
a third curved surface section (C3) with a third radius (R3) of curvature contacting the bearing surface (201, 202) during flexion between the second degree (α2) of flexion and a larger third degree (α3) of flexion,
wherein a ratio (R2/R3) of the second radius (R2) of curvature to the third radius (R3) of curvature decreases, preferably strictly, monotonically across increasing size (F1 to F9) of the femoral components (100).

15. The posterior stabilized knee prosthesis system (1) according to claim 14, wherein the ratio (R2/R3) of the second radius (R2) of curvature to the third radius (R3) of curvature decreases in the range of 1.031 to 1.019.

16. The posterior stabilized knee prosthesis system (1) according to claim 14 or 15, wherein the condyle surface (101, 102) of each femoral component (100) has
a fourth curved surface section (C4) with a fourth radius (R4) of curvature contacting the bearing surface (201, 202) during flexion between the third degree (α3) of flexion and a larger fourth degree (α4) of flexion,
wherein a ratio (R3/R4) of the third radius (R3) of curvature to the fourth radius (R4) of curvature decreases, preferably strictly, monotonically across increasing size (F1 to F9) of the femoral components (100).

17. The posterior stabilized knee prosthesis system (1) according to claim 16, wherein the ratio (R3/R4) of the third radius (R3) of curvature to the fourth radius (R4) of curvature decreases in the range of 1.059 to 1.036.

18. The posterior stabilized knee prosthesis system (1) according to claim 16 or 17, wherein the condyle surface (101, 102) of each femoral component (100) has
a fifth curved surface section (C5) with a fifth radius (R5) of curvature contacting the bearing surface (201, 202) during flexion between the fourth degree (α4) of flexion and a larger fifth degree (α5) of flexion,
wherein a ratio (R4/R5) of the fourth radius (R4) of curvature to the fifth radius (R5) of curvature decreases, preferably strictly, monotonically across increasing size (F1 to F9) of the femoral components (100).

19. The posterior stabilized knee prosthesis system (1) according to claim 18, wherein the ratio (R4/R5) of the fourth radius (R4) of curvature to the fifth radius (R5) of curvature decreases in the range of 1.020 to 1.012.

20. The posterior stabilized knee prosthesis system (1) according to claim 18 or 19, wherein a ratio (R1/R5) of the first radius (R1) of curvature to the fifth radius (R5) of curvature decreases, preferably strictly, monotonically in the range of 1.537 to 1.326.

21. The posterior stabilized knee prosthesis system (1) according to any of claims 11 to 20, wherein the cam (104) initially engages the post (203) at a degree of flexion between 35° and 60°, preferably between 45° and 60°.

## Patentansprüche

1. Posterior stabilisiertes Knieprothesensystem (1), umfassend:
einen Satz (10) Femurkomponenten (100) unterschiedlicher Größen (F1 bis F9), die zum Anbringen an distalen Femora unterschiedlicher Größen ausgestaltet sind, wobei jede Femurkomponente (100) ein Paar beabstandeter Kondylen (111, 112) aufweist, die eine Fossa intercondylica (103) dazwischen definieren und einen hinteren Nocken (104) aufweisen, der in der Fossa intercondylica (103) positioniert ist, wobei mindestens einer der Kondylen (111, 112) eine Kondylenfläche (101, 102) aufweist, die in der Sagittalebene (E) mit mehreren mindestens im Wesentlichen tangentialen Krümmungsradien (R0, R1 bis R5) gekrümmt ist,
und einen Satz (20) Tibiakomponenten (200) unterschiedlicher Größen (T1 bis T9), die zum Anbringen an proximalen Tibiae unterschiedlicher Größen (T1 bis T9) ausgestaltet sind, wobei jede Tibiakomponente (200) eine Lagerfläche (201, 202) aufweist, die in der Sagittalebene (E) mit mehreren mindestens im Wesentlichen tangentialen Krümmungsradien (AR, PR) gekrümmt ist, sowie einen Pfosten (203), der sich von der Lagerfläche (201, 202) nach oben erstreckt,
wobei jede Größe (F1 bis F9) der Femurkomponente mit mindestens einer Größe (T1 bis T9) der Tibiakomponente in Eingriff bringbar ist, um sich durch Kontakt zwischen der Kondylenfläche (101, 102) und der Lagerfläche (201, 202) und/oder durch Kontakt zwischen dem Nocken (104) und dem Pfosten (203) gelenkig zu bewegen,
**dadurch gekennzeichnet, dass** die Krümmungsradien (R0, R1 bis R5) der Kondylenfläche (101, 102) jeweils monoton über eine zunehmende Größe (F1 bis F9) der Femurkomponenten (100) zunehmen und dass die Krümmungsradien (AR, PR) der Lagerfläche (201, 202) jeweils monoton über eine zunehmende Größe (T1 bis T9) der Tibiakomponenten (200) zunehmen.

2. Posterior stabilisiertes Knieprothesensystem (1) nach Anspruch 1, wobei die Zunahme der Krümmungsradien (R0, R1 bis R5) der Kondylenfläche (101, 102) über eine zunehmende Größe (F1 bis F9) der Femurkomponenten (100) streng monoton ist und/oder dass die Zunahme der Krümmungsradien (AR, PR) der Lagerfläche (201, 202) über eine zunehmende Größe (T1 bis T9) der Tibiakomponenten (200) streng monoton ist.

3. Posterior stabilisiertes Knieprothesensystem (1) nach Anspruch 1 oder 2, wobei die Krümmungsradien (R0, R1 bis R5) der Kondylenfläche (101, 102) jeweils linear über eine zunehmende Größe (F1 bis F9) der Femurkomponenten (100) zunehmen und/oder dass die Krümmungsradien (AR, PR) der Lagerfläche (201, 202) jeweils linear über eine zunehmende Größe (T1 bis T9) der Tibiakomponenten (200) zunehmen.

4. Posterior stabilisiertes Knieprothesensystem (1) nach einem der vorhergehenden Ansprüche, wobei die Kondylenfläche (101, 102) jeder Femurkomponente (100) einen Femurverweilpunkt (FDP) aufweist, wobei ein Anterior-Posterior-Abstand (DDF) zwischen dem Femurverweilpunkt (FDP) und einem anterioren Rand (AEF) der Kondylenfläche (101, 102) über eine zunehmende Größe (F1 bis F9) der Femurkomponenten (100), vorzugweise streng, monoton zunimmt.

5. Posterior stabilisiertes Knieprothesensystem (1) nach Anspruch 4, wobei der Anterior-Posterior-Abstand (DDF) zwischen dem Femurverweilpunkt (FDP) und dem anterioren Rand (AEF) der Kondylenfläche (101, 102) linear über eine zunehmende Größe (F1 bis F9) der Femurkomponenten (100) zunimmt.

6. Posterior stabilisiertes Knieprothesensystem (1) nach Anspruch 4 oder 5, wobei der Anterior-Posterior-Abstand (DDF) zwischen 55% und 65%, vorzugsweise 60%, einer Anterior-Posterior-Gesamtabmessung (APF) der jeweiligen Femurkomponente (100) beträgt.

7. Posterior stabilisiertes Knieprothesensystem (1) nach einem der vorhergehenden Ansprüche, wobei die Lagerfläche (201, 202) jeder Tibiakomponente (200) einen Tibiaverweilpunkt (TDP) aufweist, wobei ein Anterior-Posterior-Abstand (DDF) zwischen dem Tibiaverweilpunkt (FDP) und einem anterioren Rand (AET) der Lagerfläche (201, 202) über eine zunehmende Größe (T1 bis T9) der Tibiakomponenten, vorzugweise streng, monoton zunimmt.

8. Posterior stabilisiertes Knieprothesensystem (1) nach Anspruch 7, wobei der Anterior-Posterior-Abstand (DDT) zwischen dem Tibiaverweilpunkt (TDP) und dem anterioren Rand (AET) der Lagerfläche (201, 202) linear über eine zunehmende Größe (T1 bis T9) der Tibiakomponenten (200) zunimmt.

9. Posterior stabilisiertes Knieprothesensystem (1) nach Anspruch 7 oder 8, wobei der Anterior-Posterior-Abstand (DDT) zwischen 60% und 70%, vorzugsweise 65%, einer Anterior-Posterior-Gesamtabmessung (APT) der jeweiligen Tibiakomponente (200) beträgt.

10. Posterior stabilisiertes Knieprothesensystem (1) nach einem der vorhergehenden Ansprüche, wobei die mehreren mindestens im Wesentlichen tangentialen Krümmungsradien (R0, R1 bis R5) der Kondylenfläche (101, 102) jeder Femurkomponente (100) in posteriorer Richtung entlang der Kondylenfläche (101, 102), vorzugsweise streng, monoton abnehmen.

11. Posterior stabilisiertes Knieprothesensystem (1) nach einem der vorhergehenden Ansprüche, wobei die Kondylenfläche (101, 102) jeder Femurkomponente (100) aufweist:
einen ersten gekrümmten Flächenabschnitt (C1) mit einem ersten Krümmungsradius (R1), der die Lagerfläche (201, 202) während des Beugens zwischen Strecken und einem ersten Beugungsgrad (α1) kontaktiert, und
einen zweiten gekrümmten Flächenabschnitt (C2) mit einem zweiten Krümmungsradius (R2), der die Lagerfläche (201, 202) während des Beugens zwischen dem ersten Beugungsgrad (α1) und einem größeren zweiten Beugungsgrad (α2) kontaktiert.

12. Posterior stabilisiertes Knieprothesensystem (1) nach Anspruch 11, wobei ein Verhältnis (R1/R2) des ersten Krümmungsradius (R1) zu dem zweiten Krümmungsradius (R2) über eine zunehmende Größe (F1 bis F9) der Femurkomponenten (100), vorzugsweise streng, monoton abnimmt.

13. Posterior stabilisiertes Knieprothesensystem (1) nach Anspruch 12, wobei das Verhältnis (R1/R2) des ersten Krümmungsradius (R1) zu dem zweiten Krümmungsradius (R2) im Bereich von 1,380 bis 1,240 abnimmt.

14. Posterior stabilisiertes Knieprothesensystem (1) nach einem der Ansprüche 11 bis 13, wobei die Kondylenfläche (101, 102) jeder Femurkomponente (100) aufweist:
einen dritten gekrümmten Flächenabschnitt (C3) mit einem dritten Krümmungsradius (R3), der die Lagerfläche (201, 202) während des Beugens zwischen dem zweiten Beugungsgrad (α2) und einem größeren dritten Beugungsgrad (α3) kontaktiert,
wobei ein Verhältnis (R2/R3) des zweiten Krümmungsradius (R2) zu dem dritten Krümmungsradius (R3) über eine zunehmende Größe (F1 bis F9) der Femurkomponenten (100), vorzugsweise streng, monoton abnimmt.

15. Posterior stabilisiertes Knieprothesensystem (1) nach Anspruch 14, wobei das Verhältnis (R2/R3) des zweiten Krümmungsradius (R2) zu dem dritten Krümmungsradius (R3) im Bereich von 1,031 bis 1,019 abnimmt.

16. Posterior stabilisiertes Knieprothesensystem (1) nach Anspruch 14 oder 15, wobei die Kondylenfläche (101, 102) jeder Femurkomponente (100) aufweist:
einen vierten gekrümmten Flächenabschnitt (C4) mit einem vierten Krümmungsradius (R4), der die Lagerfläche (201, 202) während des Beugens zwischen dem dritten Beugungsgrad (α3) und einem größeren vierten Beugungsgrad (α4) kontaktiert,
wobei ein Verhältnis (R3/R4) des dritten Krümmungsradius (R3) zu dem vierten Krümmungsradius (R4) über eine zunehmende Größe (F1 bis F9) der Femurkomponenten (100), vorzugsweise streng, monoton abnimmt.

17. Posterior stabilisiertes Knieprothesensystem (1) nach Anspruch 16, wobei das Verhältnis (R3/R4) des dritten Krümmungsradius (R3) zu dem vierten Krümmungsradius (R4) im Bereich von 1,059 bis 1,036 abnimmt.

18. Posterior stabilisiertes Knieprothesensystem (1) nach Anspruch 16 oder 17, wobei die Kondylenfläche (101, 102) jeder Femurkomponente (100) aufweist:
einen fünften gekrümmten Flächenabschnitt (C5) mit einem fünften Krümmungsradius (R5), der die Lagerfläche (201, 202) während des Beugens zwischen dem vierten Beugungsgrad (α4) und einem größeren fünften Beugungsgrad (α5) kontaktiert,
wobei ein Verhältnis (R4/R5) des vierten Krümmungsradius (R4) zu dem fünften Krümmungsradius (R5) über eine zunehmende Größe (F1 bis F9) der Femurkomponenten (100), vorzugsweise streng, monoton abnimmt.

19. Posterior stabilisiertes Knieprothesensystem (1) nach Anspruch 18, wobei das Verhältnis (R4/R5) des vierten Krümmungsradius (R4) zu dem fünften Krümmungsradius (R5) im Bereich von 1,020 bis 1,012 abnimmt.

20. Posterior stabilisiertes Knieprothesensystem (1) nach Anspruch 18 oder 19, wobei das Verhältnis (R1/R5) des ersten Krümmungsradius (R1) zu dem fünften Krümmungsradius (R5) im Bereich von 1,537 bis 1,326, vorzugsweise streng, monoton abnimmt.

21. Posterior stabilisiertes Knieprothesensystem (1) nach einem der Ansprüche 11 bis 20, wobei der Nocken (104) den Pfosten (203) anfänglich bei einem Beugungsgrad zwischen 35° und 60°, vorzugsweise zwischen 45° und 60°, in Eingriff nimmt.

## Revendications

1. Système de prothèse de genou postéro-stabilisé (1), comprenant :
un ensemble (10) de composants fémoraux (100) de tailles différentes (F1 à F9) configurés pour être fixés à des fémurs distaux de tailles différentes, chaque composant fémoral (100) possédant une paire de condyles (111, 112) espacés définissant entre eux une encoche intercondylaire (103), et comportant une came postérieure (104) positionnée dans l'encoche intercondylaire (103), au moins un des condyles (111, 112) ayant une surface de condyle (101, 102) courbée dans le plan sagittal (E) avec de multiples rayons de courbure au moins sensiblement tangentiels (R0, R1 à R5);
et un ensemble (20) de composants tibiaux (200) de différentes tailles (T1 à T9) configurés pour se fixer sur des tibias proximaux de tailles différentes (T1 à T9), chaque composant tibial (200) ayant une surface d'appui (201, 202) courbée dans le plan sagittal (E) avec de multiples rayons de courbure (AR, Pr) au moins sensiblement tangentiels, et ayant un montant (203) s'étendant vers le haut depuis la surface d'appui (201, 202) ;
chaque taille (F1 à F9) de composant fémoral pouvant être en prise avec au moins une taille (T1 à T9) de composant tibial pour s'articuler par contact entre la surface de condyle (101, 102) et la surface d'appui (201, 202) et/ou par contact entre la came (104) et le montant (203);
**caractérisé en ce que** les rayons de courbure (R0, R1 à R5) de la surface de condyle (101, 102) augmentent chacun de façon monotone en fonction de la taille croissante (F1 à F9) des composants fémoraux (100), et **en ce que** les rayons de courbure (Ar, Pr) de la surface d'appui (201, 202) augmentent chacun de façon monotone en fonction de la taille croissante (T1 à T9) des composants tibiaux (200).

2. Système de prothèse de genou postéro-stabilisé (1) selon la revendication 1, l'augmentation des rayons de courbure (R0, R1 à R5) de la surface de condyle (101, 102) en fonction de la taille croissante (F1 à F9) des composants fémoraux (100) étant strictement monotone et/ou l'augmentation des rayons de courbure (Ar, Pr) de la surface portante (201, 202) en fonction de la taille croissante (T1 à T9) des composants tibiaux (200) étant strictement monotone.

3. Système de prothèse de genou postéro-stabilisé (1) selon la revendication 1 ou 2, les rayons de courbure (R0, R1 à R5) de la surface de condyle (101, 102) augmentant chacun linéairement en fonction de la taille croissante (F1 à F9) des composants fémoraux (100) et/ou les rayons de courbure (Ar, Pr) de la surface d'appui (201, 202) augmentant linéairement en fonction de la taille croissante (T1 à T9) des composants tibiaux (200).

4. Système de prothèse de genou postéro-stabilisé (1) selon l'une quelconque des revendications précédentes, la surface de condyle (101, 102) de chaque composant fémoral (100) ayant un point d'arrêt fémoral (FDP), une distance antérieure-postérieure (DDF) entre le point d'arrêt fémoral (FDP) et un bord antérieur (AEF) de la surface de condyle (101, 102) augmentant, de préférence strictement, de façon monotone en fonction de la taille croissante (F1 à F9) des composants fémoraux (100).

5. Système de prothèse de genou postéro-stabilisé (1) selon la revendication 4, la distance antérieure-postérieure (DDF) entre le point d'arrêt fémoral (FDP) et le bord antérieur (AEF) de la surface de condyle (101, 102) augmentant linéairement en fonction de la taille croissante (F1 à F9) des composants fémoraux (100).

6. Système de prothèse de genou postéro-stabilisé (1) selon la revendication 4 ou 5, la distance antérieure-postérieure (DDF) étant comprise entre 55 % et 65 %, de préférence 60 %, d'une dimension antérieure-postérieure totale (APF) du composant fémoral respectif (100).

7. Système de prothèse de genou postéro-stabilisé (1) selon l'une quelconque des revendications précédentes, la surface d'appui (201, 202) de chaque composant tibial (200) ayant un point d'arrêt tibial (TDP), une distance antérieure-postérieure (DDT) entre le point d'arrêt tibial (TDP) et un bord antérieur (AET) de la surface d'appui (201, 202) augmentant, de préférence strictement, de façon monotone en fonction de la taille croissante (T1 à T9) des composants tibiaux.

8. Système de prothèse de genou postéro-stabilisé (1) selon la revendication 7, la distance antérieure-postérieure (DDT) entre le point d'arrêt tibial (TDP) et le bord antérieur (AET) de la surface d'appui (201, 202) augmentant linéairement en fonction de la taille croissante (T1 à T9) des composants tibiaux (200).

9. Système de prothèse de genou postéro-stabilisé (1) selon la revendication 7 ou 8, la distance antérieure-postérieure (DDT) étant comprise entre 60 % et 70 %, de préférence 65 %, d'une dimension antérieure-postérieure totale (APT) du composant tibial respectif (200) .

10. Système de prothèse de genou postéro-stabilisé (1) selon l'une quelconque des revendications précédentes, les multiples rayons au moins sensiblement tangentiels (R0, R1 à R5) de courbure de la surface de condyle (101, 102) de chaque composant fémoral (100) diminuant, de préférence strictement, de façon monotone dans la direction postérieure le long de la surface de condyle (101, 102).

11. Système de prothèse de genou postéro-stabilisé (1) selon l'une quelconque des revendications précédentes, la surface de condyle (101, 102) de chaque composant fémoral (100) ayant
une première section de surface courbée (C1) avec un premier rayon de courbure (R1) en contact avec la surface d'appui (201, 202) pendant la flexion entre l'extension et un premier degré (α1) de flexion, et
une deuxième section de surface courbée (C2) avec un deuxième rayon de courbure (R2) étant en contact avec la surface d'appui (201, 202) pendant la flexion entre le premier degré (α1) de flexion et un deuxième degré (α2) de flexion plus grand.

12. Système de prothèse de genou postéro-stabilisé (1) selon la revendication 11, un rapport (R1/R2) du premier rayon (R1) de courbure au deuxième rayon (R2) de courbure diminuant, de préférence strictement, de façon monotone en fonction de la taille croissante (F1 à F9) des composants fémoraux (100).

13. Système de prothèse de genou postéro-stabilisé (1) selon la revendication 12, le rapport (R1/R2) du premier rayon (R1) de courbure au deuxième rayon (R2) de courbure diminuant dans la plage de 1,380 à 1,240.

14. Système de prothèse de genou postéro-stabilisé (1) selon l'une quelconque des revendications 11 à 13, la surface de condyle (101, 102) de chaque composant fémoral (100) ayant
une troisième section de surface courbée (C3), avec un troisième rayon de courbure (R3) et en contact avec la surface d'appui (201, 202) lors de la flexion entre le deuxième degré (α2) de flexion et un troisième degré (α3) de flexion plus important,
un rapport (R2/R3) du deuxième rayon (R2) de courbure au troisième rayon (R3) de courbure diminuant, de préférence strictement, de façon monotone en fonction de la taille croissante (F1 à F9) des composants fémoraux (100).

15. Système de prothèse de genou postéro-stabilisé (1) selon la revendication 14, le rapport (R2/R3) du deuxième rayon (R2) de courbure au troisième rayon (R3) de courbure diminuant dans la plage de 1,031 à 1,019.

16. Système de prothèse de genou postéro-stabilisé (1) selon la revendication 14 ou 15, la surface de condyle (101, 102) de chaque composant fémoral (100) ayant
une quatrième section de surface courbée (C4) avec un quatrième rayon de courbure (R4) et en contact avec la surface d'appui (201, 202) lors de la flexion entre le troisième degré (α3) de flexion et un quatrième degré (α4) de flexion plus important,
un rapport (R3/R4) du troisième rayon (R3) de courbure au quatrième rayon (R4) de courbure diminuant, de préférence strictement, de façon monotone en fonction de la taille croissante (F1 à F9) des composants fémoraux (100).

17. Système de prothèse de genou postéro-stabilisé (1) selon la revendication 16, le rapport (R3/R4) du troisième rayon (R3) de courbure au quatrième rayon (R4) de courbure diminuant dans la plage de 1,059 à 1,036.

18. Système de prothèse de genou postéro-stabilisé (1) selon la revendication 16 ou 17, la surface de condyle (101, 102) de chaque composant fémoral (100) ayant
une cinquième section de surface courbée (C5) avec un cinquième rayon de courbure (R5) en contact avec la surface d'appui (201, 202) lors de la flexion entre le quatrième degré (α4) de flexion et un cinquième degré (A5) de flexion plus grand,
un rapport (R4/R5) du quatrième rayon (R4) de courbure au cinquième rayon (R5) de courbure diminuant, de préférence strictement, de façon monotone en fonction de la taille croissante (F1 à F9) des composants fémoraux (100).

19. Système de prothèse de genou postéro-stabilisé (1) selon la revendication 18, le rapport (R4/R5) du quatrième rayon (R4) de courbure au cinquième rayon (R5) de courbure diminuant dans la plage de 1,020 à 1,012.

20. Système de prothèse de genou postéro-stabilisé (1) selon la revendication 18 ou 19, un rapport (R1/R5) du premier rayon (R1) de courbure au cinquième rayon (R5) de courbure diminuant, de préférence strictement, de façon monotone dans la plage de 1,537 à 1,326.

21. Système de prothèse de genou postéro-stabilisé (1) selon l'une quelconque des revendications 11 à 20, la came (104) venant en prise initialement avec le montant (203) à un degré de flexion compris entre 35° et 60°, de préférence entre 45° et 60°.
